Europäisches Patentamt

European Patent Office

Office européen des brevets

(11) Publication number : **0 514 258 A1**

# EUROPEAN PATENT APPLICATION

(21) Application number : **92401295.8**

(51) Int. Cl.⁵ : **A61B 17/36**

(22) Date of filing : **12.05.92**

(30) Priority : **15.05.91 US 699612**

(43) Date of publication of application :
**19.11.92 Bulletin 92/47**

(84) Designated Contracting States :
**DE FR GB IT**

(71) Applicant : **S.L.T. JAPAN CO, LTD.**
**402 Ebisutei Bldg., 8, Kagurazaka 5-chome**
**Shinjuku-ku, Tokyo 162 (JP)**

(72) Inventor : **Daikuzono, Norio**
**381, Kofudai 4-chome**
**Ichihara-shi, Chiba-ken, 290-02 (JP)**

(74) Representative : **Bloch, Gérard et al**
**2, square de l'Avenue du Bois**
**F-75116 Paris (FR)**

(54) **Laser light irradiation apparatus.**

(57)    A laser light irradiation apparatus used in medical treatment while the laser light emitting surface of the apparatus is brought into contact with the living tissues. This apparatus comprises an optical fiber (10) and a holder (20) which has rigitidy higher than that of the optical fiber (10) and which surrounds the optical fiber (10) integrally. With respect to the direction extending from the output end of the optical fiber (10) toward the target area of the living tissues, laser light output end surface of the optical fiber (10) is substantially at the same level as that of themtip end (30) of the holder (20) or is projected forward from the holder (20). The above laser light output end surface is brought into contact with the target area of th eliving tissues directly. Then, the holder (20) moves so that the laser light irradiated area of the living tissues can be changed corresponding to the purpose of the medical treatment, while the holder (20) serves as a supporter or a guide, alternetively while a grip (50) connected to the holder (20) integrally serves as a supporter.

F I G. 1

This invention relates to a laser light irradiation apparatus used in medical treatments for living tissues of a human body or an animal (hereafter "living tissues of a human ..." is expressed by "tissues" or "living tissues").

Medical treatments such as incisions, evaporation, coagulation of living tissues by irradiation with laser light are conspicuous due to its ability.

Conventionally, a laser light irradiation apparatus for surgical treatments had a metal cylindrical holder supporting an optical fiber therein. The optical fiber was inserted in the holder, while its forward end is not projected from the holder. The proximal end of the optical fiber was connected to a laser light generator (laser light source). The laser light from this generator was transmitted through the optical fiber. Then, the laser light was emitted from the output end of the optical fiber so as to be irradiated against the living tissues.

In this case, the tip end of the holder was brought out of contact with the tissue-surface. Accordingly, the output end of the optical fiber was also brought out of contact with the tissue-surface. Under this condition, the laser light was irradiated against the tissue-surface in order to carry out a medical operation, which is called as a non-contact irradiation method.

In this non-contact irradiation method, since the output end of the optical fiber was brought out of contact with the laser light irradiated area of the tissues, when the angle of the holder with respect to the tissue-surface was changed even slightly, the laser light irradiated area was displaced vigorously from the actual target area. Thus, in this non-contact method, laser light irradiation could not be performed correctly. Further, the laser light emitted from the output end of the optical fiber was diffused in the air. Accordingly, in order to irradiate the required amount of laser light against the tissues, the laser light generator should have high power. Therefore, the size of the laser light irradiation apparatus should be large resulting in expensive fee for producing this apparatus.

In order to overcome these problems, the present inventor proposed a contact-method with a laser light irradiation apparatus. In this apparatus, the output end of an optical fiber was connected to a contact-tip fabricated from a ceramic material. The laser light was fed into the back end of the contact-tip from the output end of the optical fiber. Subsequently, the laser light was fed through the contact-tip and emitted from its emitting surface. Then, the medical operation was carried out with the laser light irradiation while the emitting surface of the contact-tip was brought into contact with the target area of the living tissues. This method can be used efficiently. Particularly, even if the power level of the laser light generator is not so high, higher energy can be supplied to the living tissues, comparing the conventional apparatus. Further, the laser light irradiated area of the tissues was not displaced from the actual target area. By these advantages, this contact-method has actually been put to practical use in the medical treatment.

On the other hand, when this contact method is applied, the optical fiber is required to connect to the contact-tip optically. There are several apparatuses supporting the optical fiber and the contact-tip. There can be mentioned typically apparatuses disclosed in USP Nos. 4,592,353 and 4,736,743 by the present inventor. In each apparatus, the optical fiber and the contact-tip are connected integrally by using a connector. This connector is set in a holder. This holder is operated by a medical operator so as to change the laser light irradiated area corresponding to the purpose of each medical treatment. In a surgical treatment, the operator has the holder in his hand and operates it. On the other hand, in an internal treatment, the operator moves it forward and backward outside of the body on the basis of observation with an endoscope.

However, in the contact irradiation method with the above conventional apparatus is used, there is a small gap between the output end of the optical fiber and the back end of the contact-tip. Accordingly, energy loss of the laser light is generated in this gap. In addition, since the back end of the contact-tip is heated due to the laser light irradiation, a passage or a device should be provided for supplying cool water or cool gas obtained outside of the apparatus. Thus, a sheath is provided so as to surround the optical fiber coaxially for letting the cool water or the cool gas pass therethrough. The sheath is inserted through a probe of the endoscope. Therefore, the outer diameter of the sheath as well as that of the probe of the endoscope is large. This is a severe problem in the internal treatment. Moreover, this conventional apparatus is required to have at least the optical fiber, the contact-tip and the connector connecting them integrally. Precisely, it is difficult to attain low cost due to this structure.

It is therefore the first object of the present invention to provide a laser light irradiation apparatus by which laser light can be irradiated without energy loss.

It is the second object of the present invention to provide a laser light irradiation apparatus which is suitable for forming the through-hole of an endoscope due to small outer diameter of the apparatus.

It is another object of the present invention to provide a laser light irradiation apparatus for which producing cost is low due to simple structure of the apparatus.

In order to solve these problems and attain the above objects, the present invention features a laser light irradiation apparatus used in medical treatment while the laser light emitting surface of the apparatus is brought into contact with living tissues, the apparatus comprising:

an optical fiber, the fore end portion of which is inserted in a holder so as to be buried therein, while there is no gap between at least its output end surface and the holder; and

the holder which surrounds the optical fiber integrally, at least laser light emitting portion of which is fabricated from a laser light penetrating material, which is brought into contact with the laser light irradiated area of the living tissues and which moves so that the laser light irradiated area of the living tissues can be changed corresponding to the purpose of the medical treatment, while the holder serves as a supporter or a guide, alternatively while a grip connected to the holder integrally serves as a supporter. Further, for a medical operation using an endoscope in an internal treatment, the holder can be served as a guide for the probe of the endoscope.

In the prior art, the laser light emitted from the optical fiber is fed into the back end of the contact-tip, which is brought out of contact with the output end of the optical fiber. Then, the laser light is emitted from the emitting surface of the contact-tip. However, in the apparatus of the present invention, the output end of the optical fiber is inserted in the holder so as to be buried therein without any gap. Accordingly, the laser light can be fed into the holder without power loss. Further, since the laser light is irradiated while the holder is brought into contact with the target area of the living tissues, the power loss is not produced there. Thus, even if the power of the laser light generator is low to some degree, the medical operation such as incisions can be performed effectively.

The optical fiber basically has a character as an advantage that its diameter is small. Accordingly, the output end of the optical fiber can not be molded optionally for the purpose of obtaining a wide angle of the laser light emission. Thus, in many cases, a laser light irradiated area can not be enlarged so easily at once.

As opposed to this, when the laser light is irradiated against the tissues via the holder, the laser light can be emitted widely by molding the emitting surface of the holder optionally. That is to say, the laser light can be emitted from the wide area of the emitting surface. Precisely, the laser light can be irradiated against the large area of the tissues.

According to the present invention, the optical fiber is supported by the holder. Therefore, comparing the prior art where the optical fiber is brought into contact with the target area of the living tissues directly, the emitting portion of this apparatus is more rigid. Then, even if the mechanical reaction force such as bending force is applied, its damage can be prevented.

Preferably, the holder comprises a cylindrical holder body and a transmitting tip located at its fore end portion and filled with a laser light penetrating material. In this case, the holder body can be fabricated from a material having enough strength such as a rigid metal. Accordingly, the holder can be proof against the mechanical external strength. On the other hand, when the holder consists from one unit, the material of this unit must be both laser light translating and heat resistant. However, such kind of material is limited to glass, transparent ceramics and the like. Further, the material can not be proof against the external force and can not be molded optionally, then, the producing cost becomes high. As a result, when the holder is produced while the cylindrical holder body and the transmitting tip are provided individually for the purpose of sharing function among the holder body and the transmitting tip, a low cost and easy molding can be attained.

When laser light scattering particles are dispersed in the above mentioned transmitting tip or the holder fabricated from one material, the laser light can be irradiated against the wide area of the tissues at once. In addition to the laser light scattering particles, if laser light absorbing particles are dispersed, laser light irradiation energy can be uniform in a certain scope.

A temperature detector can be provided in this apparatus. In this case, the tip end of the temperature detector is brought into contact with the external side surface of the optical fiber at its portion which is inserted so as to be buried in the holder and the lead wire of the detector is passed through between the optical fiber and the holder so as to be projected out. Thus, the temperature of the laser light irradiated area can be known on the basis of temperature-signal of the detector, because the temperature of the laser light irradiated area is proportional to the temperature at the tip end of the detector. Then, the temperature of the laser light irradiated area can be controlled. Further, by the above mentioned temperature-signal of the detector, also a specific spectra produced by melting of the holder can be detected. Therefore, by controlling the output power level of the laser light generator on the basis of the temperature-signal, the holder can be prevented from melting.

Further objects and advantages of the present invention will be apparent from the following description, reference being had to the accompanying drawing wherein preferred embodiments of the present invention are clearly shown.

Figures show irradiation apparatuses of the present invention respectively.

Fig. 1 is a schematic illustration showing an apparatus of the first embodiment in the present invention;

Fig. 2 is a schematic illustration showing an apparatus of another embodiment;

Fig. 3 is a schematic illustration showing an apparatus of still another embodiment;

Figs. 4 and 5 are schematic illustrations showing producing steps for the irradiation apparatus of Fig. 3 respectively;

Figs. 6 and 7 are schematic illustrations showing apparatuses provided with grips respectively;

Figs. 8 and 9 are schematic illustrations showing apparatuses provided with different shaped emitting portions respectively;

Figs. 10 to 14 are perspective views showing different shaped apparatuses respectively;

Figs. 15 to 17 are schematic illustrations showing apparatuses provided with the different shaped fore end portions of transmitting tips respectively;

Fig. 18 is a schematic illustration showing a laser light irradiation apparatus provided with a temperature detector.

Now, the present invention is described more particularly with the following preferred embodiments.

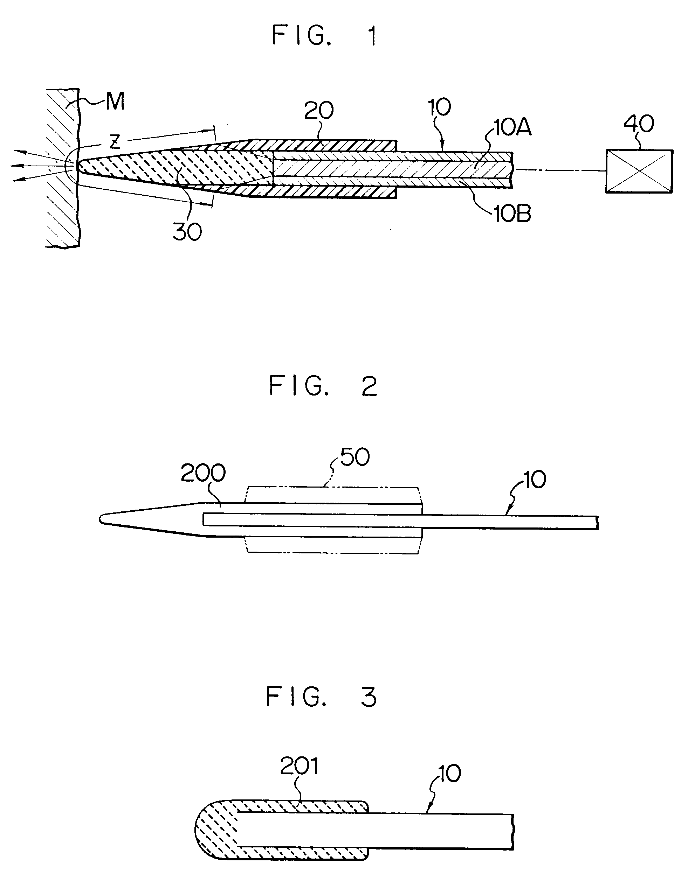

Fig. 1 shows the first embodiment. An optical fiber 10 is provided with a core 10A and a clad 10B. Then, the fore end portion of the optical fiber 10 is surrounded by a cylindrical holder 20 integrally. A laser light penetrating transmitting tip 30 is connected to the output end of the optical fiber 10. There is no gap between the back end of the transmitting tip 30 and the output end of the optical fiber 10. The transmitting tip 30 has a substantial conical shaped emitting portion with a rounded tip end and a cylindrical shaped proximal portion. The cylindrical proximal portion of the transmitting tip 30 is continued to the holder 20 external side surface of which is tapered with the same angle as that of the transmitting tip 30.

The fore end portion of the optical fiber 10 is inserted in the holder 20 and the transmitting tip 30 so as to be buried therein. Thus, it is understood that the transmitting tip 30 can transmit the laser light. The holder 20 can be fabricated from a synthetic material or from a metal material. The holder 20 preferably has flexure rigidity higher than that of the optical fiber 10. The transmitting tip 30 is fabricated from a laser light penetrating material such as quartz, artificial or natural sapphire and the like.

The fore end portion of the optical fiber 10 shown in Fig. 1 has a conical shape with a rounded tip end. Then, the rest of the optical fiber 10; its portion extended from the back end of its above conical shaped portion is covered with the holder 20. The length of the holder 20 is determined optionally depending on the usage of the apparatus, particularly, whether the apparatus is used for a surgical treatment or an internal treatment. In general, when the apparatus is used in the surgical treatment, since a medical operator has the holder in his hand, its length is preferably longer than his hand. In the internal treatment, for example, the apparatus is inserted in the through-hole of the probe of an endoscope. As long as for the purposes of the provision of the holder that the optical fiber 10 must be inserted in the through-hole surely and that the optical fiber 10 and the through hole must be provided coaxially, the holder 20 is not generally required to be long.

A laser light generator 40 is connected optically to the proximal portion of the optical fiber 10. Thus, the laser light fed from the laser light generator 40 is fed into the optical fiber 10 from its back end. Subsequently, the laser light goes through the optical fiber 10 and through the transmitting tip 30 connected to the output end of the optical fiber 10 without any gap. Finally, the laser light is emitted from the exposed emitting surface Z of the transmitting tip 30. In the surgical treatment, the medical operator holds the holder 20 in his hand and moves the holder 20 for incisions and the like while the emitting surface Z is brought into contact with the target area of the living tissues M of the human body.

The emitting surface Z of the transmitting tip 30 can be molded easily by cutting work. Precisely, a bar shaped laser light penetrating material having the same diameter throughout all its length is molded by cutting its fore end portion so as to be conical shaped. In order that the holder 20 surrounds the optical fiber 10 integrally, the following methods can be applied. As the first method, the fore end portion of the optical fiber 10 is inserted in the cylindrical holder 20 under pressure. As the second method, the optical fiber 10, the outer diameter of which is smaller than the inner diameter of the holder 20, is prepared. Then, this optical fiber 10 is inserted into the holder 20. Continuously, the holder 20 is heated outside so that its inner surface is melted to be sticked to the external surface of the optical fiber 10. As the third method, the optical fiber 10 is inserted into the holder 20 in the same way as the second method. Then, adhesive is applied on the external surface of the optical fiber 10 or on the inner surface of the holder 20 so that they can be sticked each other without heating and melting. Alternatively, when the holder 20 is fabricated from the metal, it is fixed to the external surface of the optical fiber 10 under pressure so that the optical fiber 10 can be fixed.

The transmitting tip 30 and the holder 20 can be attached each other integrally like the above methods. When the adhesive is used for this attachment, the adhesive must be capable of laser light penetrating. Alternatively, the transmitting tip 30 can be connected to the optical fiber 10 integrally. First, the material of the transmitting tip 30 is melted in a longitudinal specific mold (not shown) of the transmitting tip 30. Next, the output end of the optical fiber 10 is brought into contact with the upper surface of the material in the mold or the predetermined length of the fore end portion of the optical fiber 10 is immersed into the material. In the both cases, the melting point of the optical fiber 10 must be higher than that of the material. When the material is solidified, the integrally attaching can be finished.

In the embodiment shown in Fig. 1, a holder member comprises the cylindrical holder 20 consisting mainly the holder member and the transmitting tip 30 serving as a laser light transmitting portion. However, in the next embodiment, as shown in Fig. 2, a holder 200 consists from one unit and surrounds the whole external surface of the fore end portion of the optical fiber 10 including its output end surface and external side surface of its fore end portion. In this case, the holder 200 is fabricated from a laser light penetrating material such as heat resistant synthetic resin, ceramic and the like.

The holder 200 must be also attached to the optical fiber 10 integrally. For this attachment, there are also several methods. As the first method, laser light penetrating adhesive is used. In the second method, after the material of the holder 200 is melted in a longitudinal specific mold (not shown) of the holder 200, the predetermined length of the fore end portion of the optical fiber 10 is immersed into the melted material. In the third method, the fore end portion of the optical fiber 10 may be inserted into the molded holder 200 under pressure.

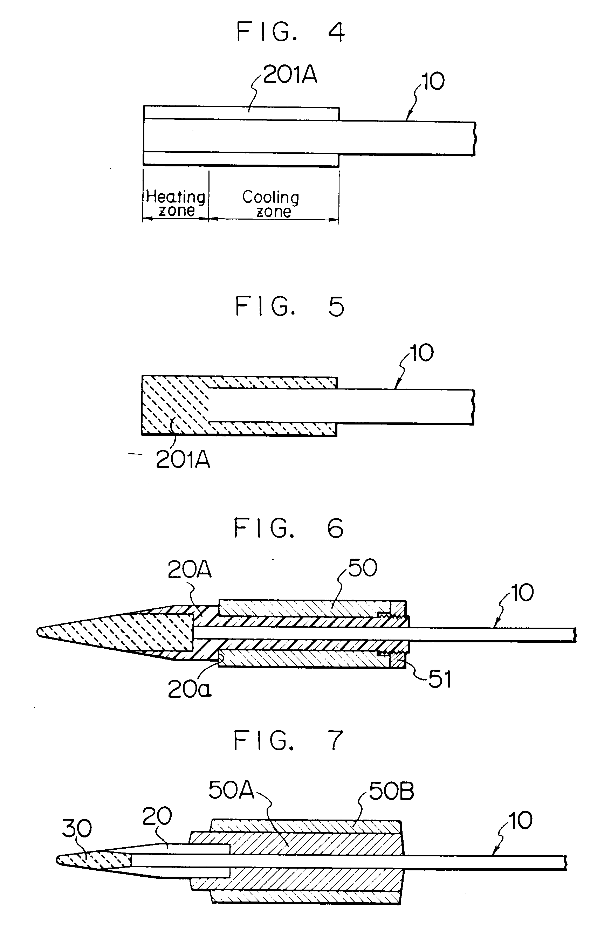

As shown in Fig. 3, a holder 201 surrounds the optical fiber 10 integrally without a boundary line. As shown in Fig. 4, a cylindrical original holder 201A is fitted so as to surround the optical fiber 10. In this case, the holder 201A has the same or almost the same melting point as that of the optical fiber 10. Then, the fore end portion of the holder 201A is heated so as to be melted while the proximal portion of the holder 201A is cooled. Thus, as shown in Fig. 5, there is no boundary line between the holder 201A and the output end surface of the optical fiber 10. The resultant holder can be utilized as it is. Alternatively, as shown in Fig. 3, before utilizing, the external surface of the holder 201A can be molded optionally with a machine work.

When the laser light irradiation apparatus is used in the surgical treatment, since the outer diameter is too small, it is difficult for a medical operator to hold the apparatus in his hand as it is. Therefore, if desired, as shown by the image line of Fig. 2, a suitable shaped grip 50 can be provided along the back end portion of the holder 20. This grip 50 can be fabricated from a synthetic material. In this case, the grip 50 is attached to the holder 20 integrally by welding. Alternatively, as shown in Fig. 6, a step 20a is formed by cutting work of a holder 20A. then, the grip 50 is fitted in the step 20a by means of nuts 51, which are applied so as to push the grip 50 forward. Thus, the grip 50 can be attached to the holder 20A integrally and mechanically.

For example, when the target area of the tissues is located deeply in the human body, the medical treatment is carried out with a laser light irradiation apparatus provided with a long holder 20. In this case, if the holder 20 is fabricated from the synthetic material, it often bends resulting in that a laser light irradiated area is displaced from the actual target area of the tissues. Therefore, as shown in Fig. 7, for example, a metal grip 50A can be passed from the back end of the holder 20 to the backward projecting portion of the optical fiber 10 from the holder 20, in order to support them simultaneously. Thus, the bending of the holder can be prevented by holding the holder via the grip 50A. Further, the grip 50A can be surrounded partly by a cover 50B made from rubber for the prevention of slip.

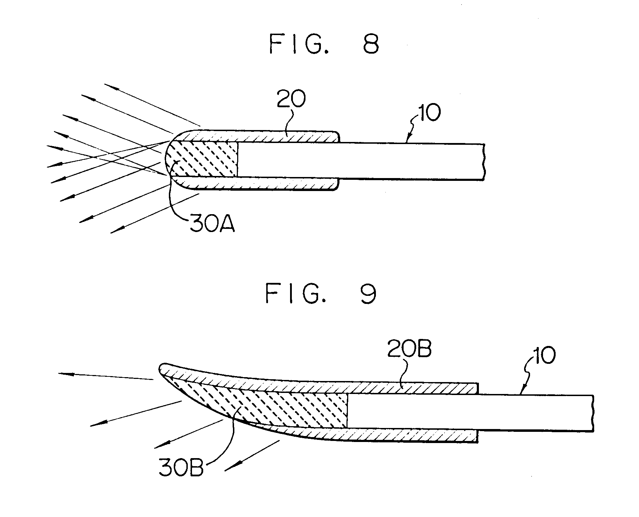

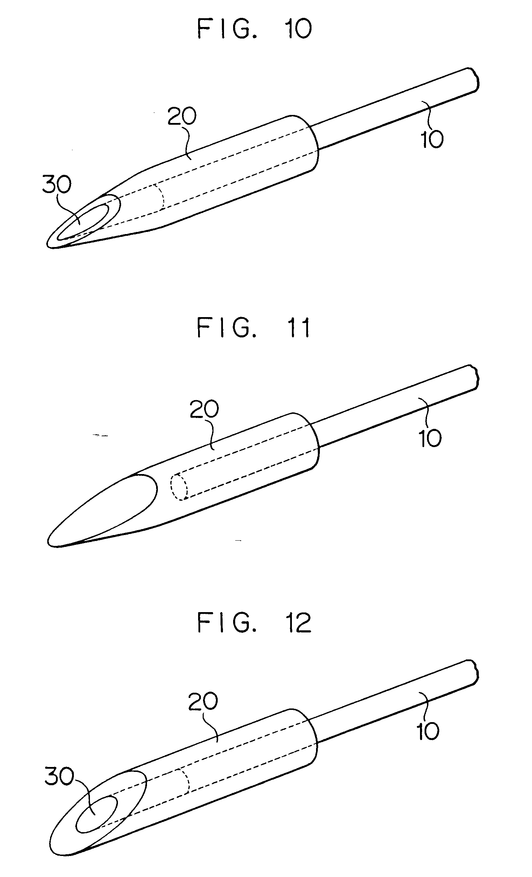

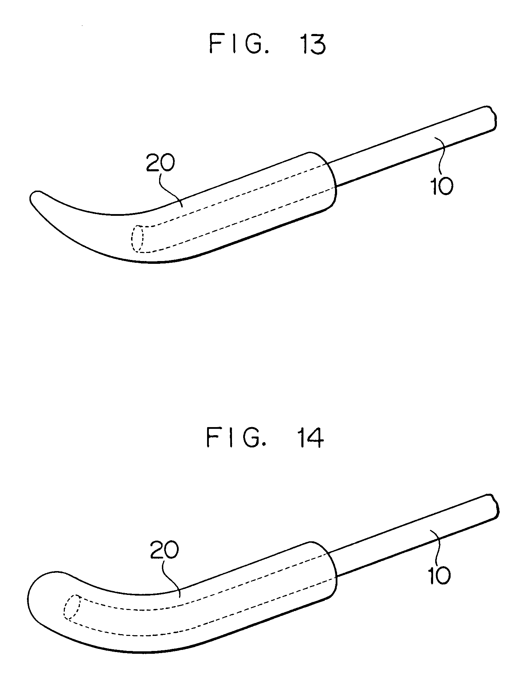

Fig. 8 shows an apparatus where the tip end of a transmitting tip 30A is rounded in the same manner as the embodiment shown in Fig. 3. Fig. 9 shows an apparatus where a transmitting tip 30B and a holder 20B are bent and its emitting portion is cut at an angle. As considered from these embodiments, in the present invention, the shapes of the transmitting tip and the holder can be selected optionally. Further, as shown in Figs. 10, 11, 12, 13 and 14 show conical chisel shaped holder, chisel shaped holder, prism shaped holder, conical curved holder, round curved holder can be molded respectively.

For connecting the above transmitting tip 30 to the output end of the optical fiber 10, molecular adhesion can be performed. Precisely, the back end of the transmitting tip 30 and the output end of the optical fiber 10 are polished respectively. Then, the above ends are connected each other without adhesive.

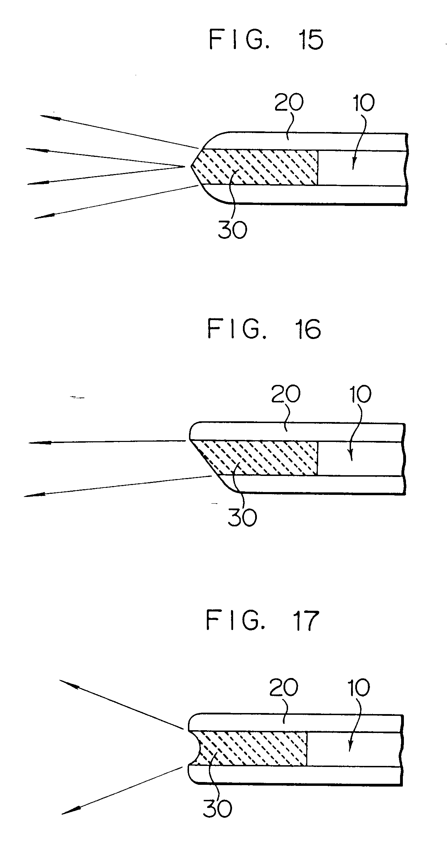

Depending on the shape of the output end portion of the optical fiber, the direction and scope of the laser light irradiation are determined. When the optical fiber has the conical shaped output end portion with rounded tip end like the shape of the transmitting tip in Fig. 1, most of the laser light is emitted forward from its tip end concentratedly. However, the laser light is slightly emitted also from the side surface of the conical shaped output end portion. As shown in Fig. 15, when its output end portion has a conical shape tapered not sharply, the direction of the laser light emission is divided upward and downward while there is few energy along its axis. As shown in Fig. 16, when its output end portion is cut at an angle, the direction of the laser light emission is altered from the axis. As shown in Fig. 17, when its output end portion has a concave at its tip end, the laser light emission has a large angle. As a result, the output end portion of the optical fiber can be shaped optionally corresponding to the purpose of each medical treatment.

In order that the laser light is irradiated uniformly against the wide area of the tissues, laser light scattering layer can be provided on the above emitting surface Z, alternatively, the emitting surface Z can be formed to be rough. The scattering layer preferably contains not only laser light scattering particles but also laser light absorbing particles. As the laser light absorbing particle, there can be mentioned typically as carbon particle, brown iron oxide and the like. On the other hand, as the laser light scattering particle, there can be mentioned typically as sapphire particle, quartz particle and the like.

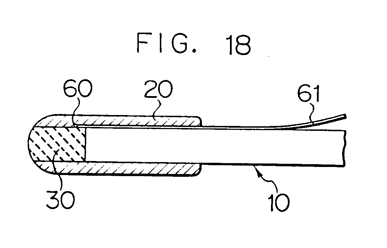

Further, as shown in Fig. 18, a temperature detector 60 such as a thermocouple can be provided, while its tip end is brought into contact with the external side surface of the optical fiber 10. A lead wire 61 of the temperature detector 60 is passed through between the optical fiber 10 and the holder 20 so as to be projected out. The projected lead wire 61 is connected to a laser light controller such as a laser light generator 40. Then, the temperature of the laser light irradiated area of the tissues can be known on the basis of the temperature-signal of the detector 60, because the temperature of the laser light irradiated area is proportional to the temperature at the tip end of the detector 60. Thus, the temperature of the laser light irradiated area can be controlled by adjusting operation with the output adjusting device of the laser light generator 40. Further, by the above mentioned temperature-signal of the detector 60, specific spectra produced by melting of the holder 20 can be detected. Therefore, by controlling the output power level of the laser light generator on the basis of the temperature-signal, the holder can be prevented from melting.

While preferred embodiments have been described, it is apparent that the present invention is not limited to the specific embodiments thereof.

**Claims**

1. A laser light irradiation apparatus used in medical treatment while the laser light emitting surface of said apparatus is brought into contact with living tissues, said apparatus comprising :

    an optical fiber (10), the fore end portion of which is inserted in a holder (20) so as to be buried therein, while there is no gap between at least its output end surface and said holder (20) ; and

    said holder (20) which surrounds said optical fiber (10) integrally, at least laser light emitting portion of which is fabricated from a laser light penetrating material, which is brought into contact with the laser light irradiated area of said living tissues and which moves so that the laser light irradiated area of said living tissues can be changed corresponding to the purpose of said medical treatment, while said holder (20) serves as a supporter or a guide, alternatively while a grip (50) connected to said holder (20) integrally serves as a supporter.

2. An apparatus according to claim 1, wherein said holder comprises a cylindrical holder body and a transmitting tip (30) located at its fore end portion and filled with a laser light penetrating material while the fore end portion of said optical fiber (10) is inserted into the hollow of said holder body (20) and the output end of said optical fiber (10) is connected to the back end of said transmitting tip (30) substantially without gap.

3. An apparatus according to claim 2, wherein laser light scattering particles are dispersed in said transmitting tip (30).

4. An apparatus according to claim 2, wherein laser light scattering particles and laser light absorbing particles are dispersed in said transmitting tip (30).

5. An apparatus according to claim 1, wherein the whole of said holder is fabricated from a laser light penetrating material and the output end of said optical fiber (10) is inserted in said holder (20) integrally without a boundary line.

6. An apparatus according to claim 1, wherein the whole of said holder is fabricated from a laser light penetrating material, the fore end portion of said optical fiber (10) is inserted in said holder (20) so as to be buried therein and laser light scattering particles and laser light absorbing particles are dispersed in said holder (20).

7. An apparatus according to claim 1, wherein a temperature detector is provided, while its tip end is brought into contact with the external side surface of the buried portion of said optical fiber (10) in said holder (20) and its lead wire is passed through between said optical fiber (10) and said holder (20) so as to be projected out.

FIG. 1

FIG. 2

FIG. 3

FIG. 4

FIG. 5

FIG. 6

FIG. 7

FIG. 8

30A

20

10

FIG. 9

20B

30B

10

FIG. 10

FIG. 11

FIG. 12

FIG. 13

20

10

FIG. 14

20

10

FIG. 15

20    10

30

FIG. 16

20    10

30

FIG. 17

20    10

30

FIG. 18

EP 0 514 258 A1

European Patent
Office

**EUROPEAN SEARCH REPORT**

Application Number

EP 92 40 1295

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | GB-A-2 227 103 (MASAHIKO HOSHINO) <br> * abstract; figures 1,4 * | 1-7 | A61B17/36 |
| X | US-A-4 273 109 (ENDERBY) <br> * column 3, line 32 - line 39; figure 2 * | 1 | |
| X | EP-A-0 372 362 (ANDREAS MAIER) <br> * column 3, line 47 - line 52; figures 6,11 * | 1,2,5 | |
| A | EP-A-0 424 272 (SLT JAPAN) <br> * column 6, paragraph 1; figure 1 * <br> * column 10, paragraph 4 * | 3,4,6 | |
| A | WO-A-8 909 569 (HGM) <br> * page 21, paragraph 4; figure 5 * | 7 | |
| A | US-A-4 660 925 (MCCAUGHAN) | | |
| A | WO-A-8 401 905 (SYMTONIC) | | |

TECHNICAL FIELDS SEARCHED (Int. Cl.5 )

A61B

The present search report has been drawn up for all claims

| Place of search | Date of completion of the search | Examiner |
|---|---|---|
| THE HAGUE | 13 AUGUST 1992 | BARTON S. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)